# EUROPÄISCHE PATENTSCHRIFT

(11) **EP 1 910 283 B1**
(45) Veröffentlichungstag und Bekanntmachung des Hinweises auf die Patenterteilung: **26.06.2013**
(21) Anmeldenummer: 06777824.1
(22) Anmeldetag: 18.07.2006
(51) Int. Cl.: C07C 407/00, C07C 409/04, C07C 409/16

(54) **REAKTOR ZUR HERSTELLUNG VON ORGANISCHEN PEROXIDEN ÜBER DIE ZWISCHENSTUFE EINES FESTEN HYDROPEROXIDS**
REACTOR FOR PRODUCING ORGANIC PEROXIDES VIA THE INTERMEDIATE OF A SOLID HYDROPEROXIDE
REACTEUR POUR PRODUIRE DES PEROXYDES ORGANIQUES AU MOYEN D'UN PRODUIT INTERMEDIAIRE D'UN HYDROPEROXYDE SOLIDE

(30) Priorität: 01.08.2005 DE 102005036055
(43) Veröffentlichungstag der Anmeldung: 16.04.2008
(73) Patentinhaber: Evonik Degussa GmbH, 45128 Essen (DE)
(72) Erfinder: APPEL, Hans, 82377 Penzberg (DE); WEINMAIER, Josef, Helmut, 84453 Mühldorf (DE)
(74) Vertreter: Dey, Michael
(86) Internationale Anmeldenummer: PCT/EP2006/064359
(87) Internationale Veröffentlichungsnummer: WO 2007/014845

(56) Entgegenhaltungen:
- EP-A1- 0 014 802
- FR-A1- 2 806 928
- US-A- 5 210 320
- US-A1- 2005 031 530
- ULLMANN AND GERHARTZ: "Ullmann's encyclopedia of Industrial Chemistry B2" 1988, VCH , WEINHEIM , XP002412443 in der Anmeldung erwähnt Seiten 10-47 - Seiten 10-4 Abbildung 48

## Beschreibung

Die Erfindung richtet sich auf einen Reaktor zur Herstellung von organischen Peroxiden über die Zwischenstufe eines festen Hydroperoxids, sowie Verfahren zur Herstellung von organischen Peroxiden unter Verwendung dieses Reaktors.

Organische Peroxide finden technische Anwendung als Initiatoren für Polymerisationsreaktionen, für Vernetzungsreaktionen von Polymeren und für die Härtung von ungesättigten Polyesterharzen. Diese Anwendungen basieren auf dem Zerfall der Peroxide an der labilen Sauerstoff-Sauerstoff Bindung in freie Radikale. Dieser Zerfall erfolgt in Abhängigkeit der chemischen Struktur des Peroxids bei unterschiedlichen Temperaturen und mit unterschiedlicher Geschwindigkeit. Die beim Zerfall freigesetzte Wärme führt im Fall einer ungenügenden Wärmeabfuhr zu einer Selbstbeschleunigung des Zerfallsvorgangs, der in eine zumeist heftige Zersetzung mündet. So sind nicht wenige Peroxide in reiner Form oder in höherer Konzentration explosionsgefährliche Stoffe. Besonders problematisch ist dabei die Handhabung von festen Peroxiden, bei denen die Zersetzung nicht nur thermisch, sondern auch durch mechanische Beanspruchung, wie zum Beispiel durch Reibung oder durch Schlageinwirkung, eingeleitet werden kann.

Bei zahlreichen technisch interessanten organischen Peroxiden erfolgt die Herstellung über die Zwischenstufe eines festen Hydroperoxids, das zur weiteren Umsetzung von der flüssigen Reaktionsmischung abgetrennt werden muss, in der es hergestellt wurde. Ein Beispiel sind die aus US 3,117,166 bekannten Derivate des festen 2,5-Dimethylhexan-2,5-dihydroperoxids. Um diese Produkte im technischen Maßstab sicher herstellen zu können, werden Apparate und Verfahren benötigt, mit denen sich die Umsetzung unter Bedingungen durchführen lässt, unter denen das feste Hydroperoxid nur wenig mechanisch beansprucht wird.

US 5,210,320 beschreibt ein einstufiges Verfahren zur Herstellung von Derivaten von 2,5-Dimethylhexan-2,5-dihydroperoxid, bei dem die nicht umgesetzten Einsatzstoffe vom festen Hydroperoxid abgetrennt, das Hydroperoxid optional gewaschen und anschließend weiter umgesetzt wird. Als Methoden zum Abtrennen des festen Hydroperoxids sind in der Beschreibung Dekantieren, Filtrieren und Zentrifugieren genannt, wobei Dekantieren bevorzugt ist. Beispiel 2 offenbart die Herstellung von 2,5-Dimethyl-2,5-di(tert-butylperoxy)hexan in einem gerührten 500 ml Doppelmantelreaktor, wobei das feste Zwischenprodukt 2,5-Dimethylhexan-2,5-dihydroperoxid durch Dekantieren von den Einsatzstoffen und der Waschflüssigkeit abgetrennt wird und der Feststoff im Reaktor verbleibt. Eine solche Abtrennung durch Dekantieren hat jedoch den Nachteil, dass eine große Menge an Flüssigkeit im Feststoff verbleibt, so dass die Abtrennung nicht besonders wirksam ist und mehrere Waschschritte erforderlich werden. Das Dokument gibt keinen Hinweis darauf, wie eine Abtrennung des festen Hydroperoxids unter Zurückhaltung des Hydroperoxids im Reaktor technisch sicher ausgeführt werden kann.

Aus dem Stand der Technik, beispielsweise Ullmanns Encyclopedia of Industrial Chemistry, Fifth Edition, Volume B2, Seite 10-47, sind gekühlte Rührnutschen bekannt, bei denen der Boden des Reaktorbehälters als Filtrationseinheit ausgebildet ist und in denen sich nacheinander eine Reaktion und eine Filtration unter Zurückhaltung des Feststoffs durchführen lässt. Für die sichere Herstellung von organischen Peroxiden sind diese Apparate jedoch aus Sicherheitsgründen nicht geeignet, da sie sich bei einem Ausfall der Kühlung während der Reaktion nicht rasch genug entleeren lassen und deshalb die Gefahr einer selbstbeschleunigenden Reaktion im Reaktor mit katastrophalen Folgen besteht.

Aufgabe der Erfindung ist deshalb die Bereitstellung eines Reaktors, der dieses Sicherheitsproblem löst und bei der Herstellung von organischen Peroxiden über die Zwischenstufe eines festen Hydroperoxids eine bessere Abtrennung des festen Hydroperoxids von den Einsatzstoffen ermöglicht.

Gegenstand der Erfindung ist ein Reaktor zur Herstellung von organischen Peroxiden, umfassend
a) einen Behälter mit einem Behälterboden
b) einen im Behälter angeordneten Rührer
c) ein im Behälterboden angeordnetes Notablassventil zur Entleerung des Reaktors in weniger als 600 Sekunden und
d) mindestens eine im Behälterboden angeordnete Filtrationseinrichtung.

Gegenstand der Erfindung ist außerdem ein Verfahren zur Herstellung eines organischen Peroxids, umfassend die Schritte
a) Herstellen eines festen Hydroperoxids in Form einer Suspension in dem erfindungsgemäßen Reaktor,
b) Filtrieren der Suspension über die im Behälterboden angeordnete Filtrationseinrichtung unter Zurückhaltung des festen Hydroperoxids im Reaktor und
c) Umsetzen des Hydroperoxids mit einem Alkylierungsmittel, einem Acylierungsmittel oder einer Carbonylverbindung.

Der erfindungsgemäße Reaktor umfasst einen Behälter mit einem Behälterboden, wobei der Behälter vorzugsweise die Form eines stehenden Zylinders hat und der Behälterboden vorzugsweise gewölbt in Form eines sogenannten Klöpperbodens ausgeführt ist. In einer bevorzugten Ausführungsform weist der Behälter zusätzlich einen Kühlmantel auf, durch den ein Kühlmedium geleitet werden kann, um den Behälterinhalt zu kühlen.

Der erfindungsgemäße Reaktor umfasst weiterhin einen im Behälter angeordneten Rührer, mit dem sich eine im Reaktor befindliche Reaktionsmischung durchmischen lässt. Der Rührer wird dabei vorzugsweise über eine Welle von der Oberseite des Reaktors her angetrieben. Als Rührer eignen sich alle für die Durchmischung von Flüssigkeiten verwendbaren Rührer. Vorzugsweise wird ein Flügelrührer eingesetzt, dessen Flügel bis knapp über dem Behälterboden reichen, wobei der Abstand des Rührers zum Behälterbodens insbesondere so bemessen wird, dass bei der Filtration der gebildete Filterkuchen gerade nicht bis zu den Flügeln des Rührers reicht. Durch eine solche Anordnung des Rührers lässt sich einerseits die mechanische Belastung des Feststoffs bei der Filtration gering halten, andererseits kann der Feststoff auch mit geringen Mengen an Waschflüssigkeit aufgerührt und damit wirksam gewaschen werden.

Der erfindungsgemäße Reaktor umfasst außerdem ein im Behälterboden angeordnetes Notablassventil zur Entleerung des Reaktors in weniger als 600 Sekunden. Die Zeit für die Entleerung des Reaktors wird dabei erfindungsgemäß bestimmt für eine Entleerung des zu 90% mit Wasser gefüllten Reaktors bei vollständig geöffnetem Notablassventil ohne Differenz zwischen Reaktorinnendruck und Außendruck. Das Notablassventil wird vorzugsweise an der tiefsten Stelle des Behälterbodens angeordnet um den Behälter vollständig entleeren zu können. Bei der bevorzugten Ausführungsform des Behälterbodens als gewölbter Klöpperboden wird das Notablassventil vorzugsweise in der Mitte des Behälterbodens angeordnet. Das erfindungsgemäße Notablassventil wird vorzugsweise so ausgeführt, dass innerhalb kurzer Zeit eine Öffnung des Ventils über den gesamten Ventilquerschnitt erreicht werden kann, beispielsweise durch Ausführung des Notablassventils als Kugelhahn, als Klappe oder als Schieber. Mit dem Notablassventil kann der Reaktor bei einer Betriebsstörung oder einer im Reaktor einsetzenden Zersetzung eines organischen Peroxids sicher entleert werden, bevor eine selbstbeschleunigende und unkontrollierte Zersetzung des organischen Peroxids auftritt. Das Notablassventil wird vorzugsweise so ausgeführt, dass der Reaktor in weniger als 180 Sekunden, insbesondere in weniger als 60 Sekunden entleert werden kann. Durch kürzere Entleerungszeiten kann der Reaktor auch bei höheren Reaktionstemperaturen und höheren Wärmefreisetzungsraten durch eine Reaktion noch sicher betrieben werden.

Eine Öffnung des Notablassventils wird vorzugsweise über eine Temperaturmessung und/oder Druckmessung im Reaktor ausgelöst. Zweckmäßig ist auch die zusätzliche Möglichkeit der Auslösung durch den Bediener der Anlage. Zweckmäßig ist auch die Verbindung des Notablassventils mit einem Auffangbehälter, in dem der abgelassene Reaktorinhalt mit einem inerten Verdünnungsmittel, beispielsweise Wasser, verdünnt und gegebenenfalls gekühlt wird.

Der erfindungsgemäße Reaktor umfasst schließlich auch mindestens eine im Behälterboden angeordnete Filtrationseinrichtung, über die sich Flüssigkeit dem Reaktor entnehmen lässt, wobei in der Flüssigkeit vorhandener Feststoff im Reaktorinneren zurückgehalten wird. Die im Behälterboden angeordnete Filtrationseinrichtung wird vorzugsweise in Form einer oder mehrerer Fritten aus einem porösen, formbeständigen Material ausgeführt. Als Material für solche Fritten eignen sich Glas, Keramik und Metall, insbesondere Edelstahl. Filtrationseinrichtungen in Form von Fritten werden vorzugsweise so gestaltet, dass die Fritte jeweils bündig im Behälterboden angeordnet ist, das heißt die der Reaktorinnenseite zugewandte Seite der Fritte liegt bündig mit der Innenseite des Behälterbodens des Reaktors.

Die im Behälterboden angeordneten Filtrationseinrichtungen umfassen zweckmäßigerweise auch jeweils mindestens eine Leitung zur Entnahme von Flüssigkeit, die durch das Filterelement durchgetreten ist, sowie eine Absperrvorrichtung zwischen der Fritte und der Entnahmeleitung, mit der sich verhindern lässt dass Flüssigkeit unbeabsichtigt aus dem Reaktor in die Entnahmeleitung gelangt. Die Filtrationseinrichtung wird dabei vorzugsweise so gestaltet, dass das Volumen zwischen dem Filterelement und der Absperrvorrichtung möglichst klein,gehalten wird.

In einer bevorzugten Ausführungsform wird die Filtrationseinrichtung austauschbar in einer Öffnung im Behälterboden angeordnet. Die Öffnung wird dabei zweckmäßig als runde Flanschöffnung ausgebildet, mit der die Filtrationseinrichtung über Schrauben verbunden wird.

In einer weiteren bevorzugten Ausführungsform umfasst der Reaktor zusätzlich mindestens eine im Behälterboden angeordnete und in den Innenraum des Behälters ragende Kühleinrichtung. Die Kühleinrichtung wird dabei vorzugsweise so ausgeführt, dass der überwiegende Teil der Kühlfläche in der Nähe des Behälterbodens liegt, so dass eine wirksame Kühlung bei einer nur teilweisen Befüllung des Reaktors erzielt wird. Die Kühleinrichtung wird vorzugsweise als von einem Kühlmedium durchströmter Wärmetauscher ausgeführt, insbesondere als Rohrschlange, Rohrbündelwärmetauscher oder Plattenwärmetauscher. Die Kühleinrichtung kann dabei unterhalb des Rührers oder vorzugsweise neben dem Rührer angeordnet sein.

Bei der Ausführungsform des Reaktors mit austauschbar angeordneten Filtrationseinrichtungen werden vorzugsweise Kühleinrichtungen verwendet, die mit den Filtrationseinrichtungen austauschbar sind. Besonders bevorzugt werden dabei Rohrbündelwärmetauscher mit Schwimmkopf verwendet, die durch eine Öffnung im Behälterboden von unten in den Behälter eingebaut werden. In dieser Ausführungsform des Reaktors kann die Kühlleistung des Reaktors durch Austausch eines Teils der Filtrationseinrichtungen gegen Kühleinrichtungen jeweils an den Kühlbedarf der im Reaktor durchgeführten Umsetzung angepasst werden.

Der erfindungsgemäße Reaktor ermöglicht eine sichere Herstellung und weitere Umsetzung von festen Hydroperoxiden, bei der die Hydroperoxide nur geringen mechanischen Belastungen ausgesetzt werden und bei der eine im Reaktor beginnende Zersetzung des Hydroperoxids jederzeit rasch und sicher durch Entleeren des Reaktors beherrscht werden kann.

Das erfindungsgemäße Verfahren zur Herstellung eines organischen Peroxids erfolgt über die Zwischenstufe eines festen Hydroperoxids. Im ersten Schritt des Verfahrens wird dabei das feste Hydroperoxid in dem erfindungsgemäßen Reaktor in Form einer Suspension hergestellt. Die Herstellung dieser Suspension erfolgt nach bekannten Verfahren. Vorzugsweise wird als festes Hydroperoxid 2,5-Dimethylhexan-2,5-dihydroperoxid hergestellt.

Eine bevorzugte Methode zur Herstellung einer Suspension eines festen Hydroperoxids ist die Umsetzung eines tertiären Alkohols mit Wasserstoffperoxid in einem sauren Medium. Zur Herstellung von 2,5-Dimethylhexan-2,5-dihydroperoxid wird bei dieser Methode 2,5-Dimethylhexan-2,5-diol mit Wasserstoffperoxid umgesetzt, vorzugsweise unter Zusatz von Schwefelsäure. Geeignete Reaktionsbedingungen für diese Umsetzung sind aus US 3,117,166 und US 5,210,320 bekannt.

Eine weitere bevorzugte Methode zur Herstellung von 2,5-Dimethylhexan-2,5-dihydroperoxid ist die aus WO 96/03372 bekannte Umsetzung von 2,5-Dimethyl-1,5-hexadien mit Wasserstoffperoxid in einem sauren Medium. Bei dieser Ausführungsform wird der erfindungsgemäße Reaktor vorzugsweise in der Ausführungsform mit zusätzlicher Kühleinrichtung verwendet um die hohe Reaktionswärme bei der Herstellung des Hydroperoxids sicher und rasch abführen zu können.

Während der Herstellung der Suspension des festen Hydroperoxids wird vorzugsweise der Durchtritt von Flüssigkeit durch die im Reaktorboden angeordnete Filtrationseinrichtung dadurch verhindert, dass entweder eine hinter der Filtrationseinrichtung angeordnete Absperrvorrichtung geschlossen bleibt oder durch die Filtrationseinrichtung ein Gasstrom in den Reaktor geleitet wird, der dem Durchtritt von Flüssigkeit entgegenwirkt.

Im zweiten Schritt des erfindungsgemäßen Verfahrens wird dann die Suspension des festen Hydroperoxids über die im Behälterboden angeordnete Filtrationseinrichtung filtriert, wobei das feste Hydroperoxid im Reaktor zurückgehalten und die Flüssigkeit dem Reaktor entnommen wird. Die Filtration wird vorzugsweise durch Anlegen eines Unterdrucks an die Filtrationseinrichtung bewirkt, so dass die Flüssigkeit durch den Druck im Reaktor durch die Filtrationseinrichtung gedrückt wird.

Das im Reaktor zurückgehaltene feste Hydroperoxid kann zusätzlich noch mit einer Waschflüssigkeit gewaschen werden, um anhaftende Einsatzstoffe zu entfernen, bevor es weiter umgesetzt wird. Die Waschflüssigkeit wird dazu so gewählt, dass sie das Hydroperoxid nur wenig, die zu entfernenden Einsatzstoffe jedoch gut löst. Als Waschflüssigkeit für 2,5-Dimethylhexan-2,5-dihydroperoxid eignen sich Wasser, wässrige Natriumsulfatlösung oder wässrige Ammoniumsulfatlösung. Vorzugsweise wird das feste Hydroperoxid dazu in der Waschflüssigkeit suspendiert, wobei zweckmäßigerweise der im Reaktor angeordnete Rührer verwendet wird um eine gleichmäßige Suspension zu erhalten. Die erhaltene Suspension wird dann über die im Behälterboden angeordnete Filtrationseinrichtung unter Zurückhaltung des festen Hydroperoxids im Reaktor filtriert.

Im erfindungsgemäßen Verfahren wird anschließend das im Reaktor zurückgehaltene Hydroperoxid mit einem Alkylierungsmittel, einem Acylierungsmittel oder einer Carbonylverbindung zum gewünschten organischen Peroxid umgesetzt. Die für diese Umsetzung erforderlichen Reaktionsbedingungen sind aus dem Stand der Technik bekannt. Vorzugsweise wird in diesem Schritt das Hydroperoxid in einem Lösungsmittel gelöst, bevor es mit dem Alkylierungsmittel, dem Acylierungsmittel oder der Carbonylverbindung umgesetzt wird, um die bei der Umsetzung frei werdende Reaktionswärme sicher abführen zu können. Die Umsetzung mit dem Alkylierungsmittel, dem Acylierungsmittel oder er Carbonylverbindung kann dabei in dem erfindungsgemäßen Reaktor erfolgen. Alternativ kann das Hydroperoxid aber auch in einem Lösungsmittel gelöst oder suspendiert und in einen anderen Reaktor überführt werden, um dort die Umsetzung mit dem Alkylierungsmittel, dem Acylierungsmittel oder der Carbonylverbindung durchzuführen.

In einer bevorzugten Ausführungsform des erfindungsgemäßen Verfahrens wird als Alkylierungsmittel ein tertiärer Alkohol verwendet und die Umsetzung des Hydroperoxids mit dem tertiären Alkohol erfolgt in Gegenwart einer starken Säure. Als tertiärer Alkohol werden vorzugsweise tert-Butanol, tert-Amylalkohol oder Cumylalkohol eingesetzt. Geeignete Reaktionsbedingungen für diese Umsetzung sind aus US 5,210,320 bekannt. In einer besonders bevorzugten Ausführungsform des Verfahrens wird über die Zwischenstufe 2,5-Dimethylhexan-2,5-dihydroperoxid durch Umsetzung mit tert-Butanol in Gegenwart von Schwefelsäure das Produkt 2,5-Dimethyl-2,5-di(tert-butylperoxy)hexan erhalten.

In weiteren bevorzugten Ausführungsformen des erfindungsgemäßen Verfahrens wird das Hydroperoxid mit einem Acylierungsmittel aus der Reihe der Carbonsäurechloride, Carbonsäureanhydride und Chlorformiate umgesetzt. Geeignete Reaktionsbedingungen für die Umsetzungen sind aus US 3,117,166 bekannt.

Vorzugsweise wird als Acylierungsmittel ein Carbonsäurechlorid aus der Reihe Acetylchlorid, Isobutyrylchlorid, Pivaloylchlorid, 2-Ethylhexanoylchlorid, 3,5,5-Trimethylhexanoylchlorid, Neodecanoylchlorid und Benzoylchlorid eingesetzt. In einer besonders bevorzugten Ausführungsform des Verfahrens wird über die Zwischenstufe 2,5-Dimethylhexan-2,5-dihydroperoxid durch Umsetzung mit einem dieser bevorzugten Carbonsäurechloride eine der Verbindungen 2,5-Dimethyl-2,5-di(acetylperoxy)hexan, 2,5-Dimethyl-2,5-di(isobutyrylperoxy)hexan, 2,5-Dimethyl-2,5-di(pivaloylperoxy)hexan, 2,5-Dimethyl-2,5-di(2-ethylhexanoylperoxy)hexan, 2,5-Dimethyl-2,5-di(3,5,5-trimethylhexanoylperoxy)hexan oder 2,5-Dimethyl-2,5-di(benzoylperoxy)hexan hergestellt.

In einer weiteren bevorzugten Ausführungsform wird als Acylierungsmittel vorzugsweise ein Chlorformiat aus der Reihe Isopropylchlorformiat, n-Butylchlorformiat, sec-Butylchlorformiat, 2-Ethylhexylchlorformiat, Myristylchlorformiat, Cetylchlorformiat,
Cyclohexylchlorformiat oder
4-tert-Butylcyclohexylchlorformiat eingesetzt. In einer besonders bevorzugten Ausführungsform des Verfahrens wird über die Zwischenstufe 2,5-Dimethylhexan-2,5-dihydroperoxid durch Umsetzung mit einem dieser bevorzugten Chlorformiate eine der Verbindungen
2,5-Dimethyl-2,5-di(isopropyloxycarbonylperoxy)hexan, 2,5-Dimethyl-2,5-di(n-butyloxycarbonylperoxy)hexan, 2,5-Dimethyl-2,5-di(sec-butyloxycarbonylperoxy)hexan, 2,5-Dimethyl-2,5-di(2-ethylhexyloxycarbonylperoxy)hexan, 2,5-Dimethyl-2,5-di(myristyloxycarbonylperoxy)hexan, 2,5-Dimethyl-2,5-di(cetyloxycarbonylperoxy)hexan, 2,5-Dimethyl-2,5-di(cyclohexyloxycarbonylperoxy)hexan oder 2,5-Dimethyl-2,5-di(4-tert-butylcyclohexyloxycarbonylperoxy) hexan hergestellt.

In einer weiteren bevorzugten Ausführungsform wird als Carbonylverbindung Aceton eingesetzt. In einer besonders bevorzugten Ausführungsform des Verfahrens wird über die Zwischenstufe 2,5-Dimethylhexan-2,5-dihydroperoxid durch Umsetzung mit Aceton die Verbindung 3,3,6,6,9,9-Hexamethyl-cyclo-1,2,4,5-tetraoxanonan hergestellt.

Das erfindungsgemäße Verfahren zur Herstellung organischer Peroxide über die Zwischenstufe eines festen Hydroperoxids liefert Produkte mit verbesserter Reinheit, da es eine wirksamere Abtrennung des festen Hydroperoxids von den zu seiner Herstellung eingesetzten Stoffen ermöglicht. Durch die wirksamere Wäsche des festen Hydroperoxids lässt sich außerdem die zur Wäsche benötigte Menge an Waschflüssigkeit verringern, so dass bei dem erfindungsgemäßen Verfahren weniger Abfall anfällt.
Fig. 1 zeigt eine Ausführungsform des erfindungsgemäßen Reaktors.
Fig. 2 zeigt eine alternative Ausführungsform des erfindungsgemäßen Reaktors mit zusätzlicher Kühleinrichtung.

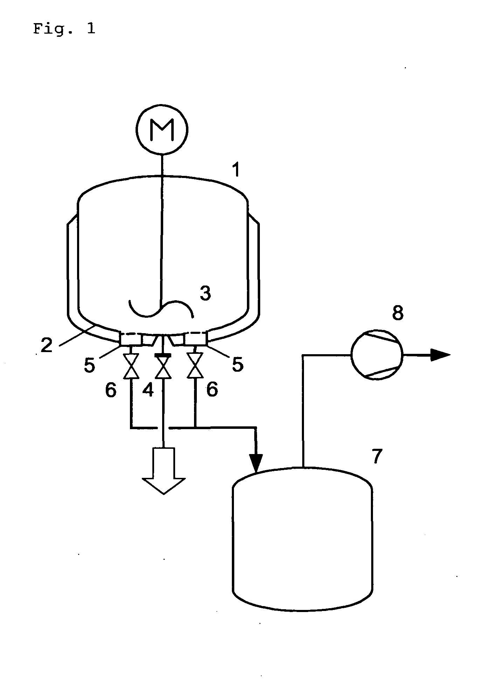

In der in Fig. 1 gezeigten Ausführungsform weist der erfindungsgemäße Reaktor einen Behälter (1) in Form eines zylindrischen Doppelmantelbehälters auf, dessen Behälterboden (2) als Klöpperboden ausgeführt ist. Der Behälter (1) weist eine Füllhöhe von 120 cm und einen Innendurchmesser von 120 cm auf. Im Behälter ist ein von oben angetriebener Rührer (3) in Form eines Impellerrührers angeordnet, dessen Flügel bis 18 cm über den Behälterboden reichen. Im Behälterboden (2) ist mittig ein Notablassventil (4) mit einem Durchmesser der Ablassöffnung von 18 cm angeordnet. Der Behälterboden weist zusätzlich noch vier Flanschöffnungen mit einem Durchmesser von jeweils 21 cm auf. In jede der Flanschöffungen ist eine Filtrationseinrichtung (5) eingebaut, die eine Metallsinterplatte über den gesamten Öffnungsquerschnitt aufweist, wobei die Metallsinterplatte im eingebauten Zustand bündig mit der Innenwand des Behälters (1) abschließt. Jede Filtrationseinrichtung ist über ein Absperrventil (6) und Verbindungsleitungen mit einem Sammelbehälter (7) für Flüssigkeit und einer Vakuumpumpe (8) verbunden. Das Volumen zwischen Metallsinterplatte und Absperrventil beträgt dabei ca. 1 1 je Filtrationseinrichtung.

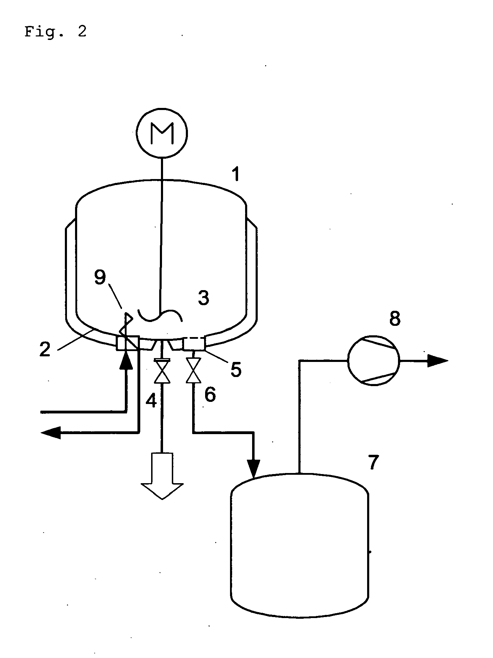

In der in Fig. 2 gezeigten Ausführungsform weist der erfindungsgemäße Reaktor eine zusätzliche im Behälterboden (2) angeordnete Kühleinrichtung (9) auf, die in den Innenraum des Behälters ragt. Die Kühleinrichtung (9) kann dabei die Form einer Rohrschlange, eines Rohrbündels oder eines Plattenwärmetauschers haben. Die Kühleinrichtung (9) ist so ausgeführt, dass sie mit der Filtrationseinrichtung (5) austauschbar ist.

### Beispiel

### Herstellung von 2,5-Dimethyl-2,5-di(tert-butylperoxy)hexan

In einem Reaktor entsprechend Fig. 1 wurde eine Mischung aus 550 kg 70 Gew.-% Wasserstoffperoxid und 366 kg 80 Gew.-% Schwefelsäure vorgelegt. Unter Rühren und Kühlen des Reaktors über den Doppelmantel wurden innerhalb 60 min 250 kg 2,5-Dimethyl-2,5-hexandiol zudosiert, wobei die Temperatur von 20°C auf 30-32 °C anstieg. Die Reaktionsmischung wurde noch 90 min bei 30°C gerührt und danach mit 200 kg Wasser versetzt. Dann wurde der Rührer abgeschaltet und die gebildete Suspension innerhalb von 10 min durch Anlegen von Vakuum an die Filtrationseinrichtungen (5) filtriert, wobei die Flüssigphase in den Sammelbehälter (7) gesaugt wurde und das ausgefallene 2,5-Dimethylhexan-2,5-dihydroperoxid im Behälter (2) zurückgehalten wurde. Das im Reaktor zurückgehaltene feste Produkt wurde durch Zugabe von 500 kg Wasser, Rühren der Suspension für 3 min und erneutes Filtrieren durch Anlegen von Vakuum an die Filtrationseinrichtungen (5) gewaschen. Dieser Waschvorgang wurde einmal wiederholt. Danach wurden 481 kg tert-Butanol zugesetzt und der Feststoff durch Rühren und Erwärmen darin gelöst. Zu der erhaltenen Lösung wurden unter Rühren und Kühlen des Reaktors über den Doppelmantel innerhalb von 60 min 500 kg 80 Gew.-% Schwefelsäure dosiert, wobei die Temperatur auf 40 bis 45°C anstieg. Die Reaktionsmischung wurde weitere 60 min bei dieser Temperatur gerührt und anschließend mit 50 kg Wasser versetzt. Die untere, wässrige Phase wurde abgetrennt und das flüssige Produkt wurde nacheinander mit 150 kg Wasser, 150 kg 1 Gew.-% Natronlauge und 150 kg Wasser gewaschen, indem die Phasen verrührt und anschließend die untere, wässrige Phase abgetrennt wurde. Nach Entfernen von restlichem Wasser und flüchtigen Nebenprodukten durch Strippen im Vakuum in einer Strippkolonne wurden 405 kg (90 %) 2,5-Dimethyl-2,5-di(tert-butylperoxy)hexan erhalten.

## Patentansprüche

1. Reaktor zur Herstellung von organischen Peroxiden, umfassend
a) einen Behälter (1) mit einem Behälterboden (2)
b) einen im Behälter angeordneten Rührer (3)
c) ein im Behälterboden angeordnetes Notablassventil (4) zur Entleerung des Reaktors in weniger als 600 Sekunden und
d) mindestens eine im Behälterboden angeordnete Filtrationseinrichtung (5).

2. Reaktor nach Anspruch 1,
**dadurch gekennzeichnet,**
**dass** er zusätzlich mindestens eine im Behälterboden (2) angeordnete und in den Innenraum des Behälters (1) ragende Kühleinrichtung (9) aufweist.

3. Reaktor nach Anspruch 1 oder 2,
**dadurch gekennzeichnet,**
**dass** die Filtrationseinrichtung (5) eine Fritte ist, die bündig im Behälterboden (2) angeordnet ist.

4. Reaktor nach einem der Ansprüche 1 bis 3,
**dadurch gekennzeichnet,**
**dass** die Filtrationseinrichtung (5) austauschbar in einer Öffnung im Behälterboden (2) angeordnet ist.

5. Reaktor nach Anspruch 4,
**dadurch gekennzeichnet,**
**dass** er zusätzlich mindestens eine im Behälterboden (2) angeordnete und in den Innenraum des Behälters (1) ragende Kühleinrichtung (9) aufweist, wobei die Kühleinrichtung (9) mit der Filtrationseinrichtung (5) austauschbar ist.

6. Verfahren zur Herstellung eines organischen Peroxids, umfassend die Schritte
a) Herstellen eines festen Hydroperoxids in Form einer Suspension in einem Reaktor gemäß einem der Ansprüche 1 bis 5,
b) Filtrieren der Suspension über die im Behälterboden (2) angeordnete Filtrationseinrichtung (5) unter Zurückhaltung des festen Hydroperoxids im Reaktor und
c) Umsetzen des Hydroperoxids mit einem Alkylierungsmittel, einem Acylierungsmittel oder einer Carbonylverbindung.

7. Verfahren nach Anspruch 6,
**dadurch gekennzeichnet,**
**dass** das feste Hydroperoxid 2,5-Dimethylhexan-2,5-dihydroperoxid ist.

8. Verfahren nach Anspruch 6 oder 7,
**dadurch gekennzeichnet,**
**dass** die Herstellung des festen Hydroperoxids durch Umsetzung eines tertiären Alkohols mit Wasserstoffperoxid in einem sauren Medium erfolgt.

9. Verfahren nach Anspruch 7,
**dadurch gekennzeichnet,**
**dass** die Herstellung von 2,5-Dimethylhexan-2,5-dihydroperoxid durch Umsetzung von 2,5-Dimethyl-1,5-hexadien mit Wasserstoffperoxid in einem sauren Medium erfolgt.

10. Verfahren nach einem der Ansprüche 6 bis 9,
**dadurch gekennzeichnet,**
**dass** das feste Hydroperoxid zwischen Schritt b) und Schritt c) in einer Waschflüssigkeit suspendiert wird und die erhaltene Suspension über die im Behälterboden (2) angeordnete Filtrationseinrichtung (5) unter Zurückhaltung des festen Hydroperoxids im Reaktor filtriert wird.

11. Verfahren nach einem der Ansprüche 6 bis 10, **dadurch gekennzeichnet,**
**dass** das Hydroperoxid in Schritt c) in einem Lösungsmittel gelöst wird bevor es mit dem Alkylierungsmittel, dem Acylierungsmittel oder der Carbonylverbindung umgesetzt wird.

12. Verfahren nach einem der Ansprüche 6 bis 11, **dadurch gekennzeichnet,**
**dass** das Alkylierungsmittel in Schritt c) ein tertiärer Alkohol ist und die Umsetzung in Gegenwart einer starken Säure erfolgt.

13. Verfahren nach Anspruch 12,
**dadurch gekennzeichnet,**
**dass** das Alkylierungsmittel ein tertiärer Alkohol aus der Reihe tert-Butanol, tert-Amylalkohol und Cumylalkohol ist.

14. Verfahren nach einem der Ansprüche 6 bis 11, **dadurch gekennzeichnet,**
**dass** das Acylierungsmittel in Schritt c) ausgewählt ist aus Carbonsäurechloriden, Carbonsäureanhydriden und Chlorformiaten.

15. Verfahren nach Anspruch 14,
**dadurch gekennzeichnet,**
**dass** das Acylierungsmittel ein Carbonsäurechlorid aus der Reihe Acetylchlorid, Isobutyrylchlorid, Pivaloylchlorid, 2-Ethylhexanoylchlorid, 3,5,5-Trimethylhexanoylchlorid, Neodecanoylchlorid und Benzoylchlorid ist.

16. Verfahren nach Anspruch 14,
**dadurch gekennzeichnet,**
**dass** das Acylierungsmittel ein Chlorforimat aus der Reihe Isopropylchlorformiat, n-Butylchlorformiat, sec-Butylchlorformiat, 2-Ethylhexylchlorformiat, Myristylchlorformiat, Cetylchlorformiat, Cyclohexylchlorformiat und 4-tert-Butylcyclohexylchlorformiat ist.

17. Verfahren nach einem der Ansprüche 6 bis 11, **dadurch gekennzeichnet,**
**dass** die Carbonylverbindung Aceton ist.

## Claims

1. Reactor for producing organic peroxides,
comprising
a) a vessel (1) with a vessel bottom (2)
b) a stirrer (3) located in the vessel
c) an emergency release valve (4) located in the vessel bottom for emptying the reactor in less than 600 seconds
and
d) at least one filtration device (5) located in the vessel bottom.

2. Reactor according to claim 1,
**characterized in that**
it additionally has at least one cooling device (9) located in the vessel bottom (2) and projecting into the interior of the vessel (1).

3. Reactor according to claim 1 or 2,
**characterized in that**
the filtration device (5) is a frit which is located flush in the vessel bottom (2).

4. Reactor according to one of the claims 1 to 3,
**characterized in that**
the filtration device (5) is located in an exchangeable manner in an opening in the vessel bottom (2).

5. Reactor according to claim 4,
**characterized in that**
it additionally has at least one cooling device (9) located in the vessel bottom (2) and projecting into the interior of the vessel (1), wherein the cooling device (9) is interchangeable with the filtration device (5).

6. Process for producing an organic peroxide,
comprising the steps
a) producing a solid hydroperoxide in the form of a suspension in a reactor according to one of the claims 1 to 5,
b) filtering the suspension through the filtration device (5) located in the vessel bottom (2) while retaining the solid hydroperoxide in the reactor and
c) reacting the hydroperoxide with an alkylating agent, an acylating agent or a carbonyl compound.

7. Process according to claim 6,
**characterized in that**
the solid hydroperoxide is 2,5-dimethylhexane-2,5-dihydroperoxide.

8. Process according to claim 6 or 7,
**characterized in that**
the solid hydroperoxide is produced by reacting a tertiary alcohol with hydrogen peroxide in an acid medium.

9. Process according to claim 7,
**characterized in that**
2,5-dimethylhexane-2,5-dihydroperoxide is produced by reacting 2,5-dimethyl-1,5-hexadiene with hydrogen peroxide in an acid medium.

10. Process according to one of the claims 6 to 9,
**characterized in that**
the solid hydroperoxide is suspended in a wash liquid between step b) and step c) and the resulting suspension is filtered through the filtration device (5) located in the vessel bottom (2) while retaining the solid hydroperoxide in the reactor.

11. Process according to one of the claims 6 to 10,
**characterized in that**
in step c) the hydroperoxide is dissolved in a solvent before it is reacted with the alkylating agent, the acylating agent or carbonyl compound.

12. Process according to one of the claims 6 to 11,
**characterized in that**
in step c) the alkylating agent is a tertiary alcohol and the reaction takes place in the presence of a strong acid.

13. Process according to claim 12,
**characterized in that**
the alkylating agent is a tertiary alcohol from the group comprising tert-butanol, tert-amyl alcohol and cumyl alcohol.

14. Process according to one of the claims 6 to 11,
**characterized in that**
in step c) the acylating agent is selected from carboxylic acid chlorides, carboxylic acid anhydrides and chloroformates.

15. Process according to claim 14,
**characterized in that**
the acylating agent is a carboxylic acid chloride from the group comprising acetyl chloride, isobutyryl chloride, pivaloyl chloride, 2-ethylhexanoyl chloride, 3,5,5-trimethylhexanoyl chloride, neodecanoyl chloride and benzoyl chloride.

16. Process according to claim 14,
**characterized in that**
the acylating agent is a chloroformate from the group comprising isopropyl chloroformate, n-butyl chloroformate, sec-butyl chloroformate, 2-ethylhexyl chloroformate, myristyl chloroformate, cetyl chloroformate, cyclohexyl chloroformate and 4-tert-butylcyclohexyl chloroformate.

17. Process according to one of the claims 6 to 11,
**characterized in that**
the carbonyl compound is acetone.

## Revendications

1. Réacteur pour la préparation de peroxydes organiques, comprenant :
a) un récipient (1) avec un fond de récipient (2) ;
b) un agitateur (3) agencé dans le récipient ;
c) une soupape de détente d'urgence (4) agencée dans le fond du récipient, pour le vidage du réacteur en moins de 600 secondes, et
d) au moins un dispositif de filtration (5) agencé dans le fond du récipient.

2. Réacteur selon la revendication 1, **caractérisé en ce qu'**il présente en outre, au moins un dispositif de refroidissement (9) agencé dans le fond du récipient (2) et dépassant dans l'espace intérieur du réacteur (1).

3. Réacteur selon la revendication 1 ou 2, **caractérisé en ce que** le dispositif de filtration (5) est un fritté, qui est agencé à niveau dans le fond du récipient (2).

4. Réacteur selon l'une des revendications 1 à 3, **caractérisé en ce que** le dispositif de filtration (5) est agencé de manière à pouvoir être changé dans une ouverture dans le fond du récipient (2).

5. Réacteur selon la revendication 4, **caractérisé en ce qu'**il présente en outre, au moins un dispositif de refroidissement (9) agencé dans le fond du récipient (2) et dépassant dans l'espace intérieur du réacteur (1), où le dispositif de refroidissement (9) peut être échangeable avec le dispositif de filtration (5).

6. Procédé de préparation d'un peroxyde organique, comprenant les étapes de :
a) préparation d'un hydroperoxyde solide sous forme d'une suspension dans un réacteur selon l'une des revendication 1 à 5,
b) filtration de la suspension par le dispositif de filtration (5) agencé dans le fond du récipient (2) avec retenue de l'hydroperoxyde solide dans le réacteur, et
c) réaction de l'hydroperoxyde avec un agent d'alkylation, un agent d'acylation ou un composé carbonylé.

7. Procédé selon la revendication 6, **caractérisé en ce que** l'hydroperoxyde solide est le 2,5-diméthyl-hexane-2,5-dihydroperoxyde.

8. Procédé selon la revendication 6 ou 7, **caractérisé en ce que** la préparation de l'hydroperoxyde solide est réalisée par réaction d'un alcool tertiaire avec le peroxyde d'hydrogène en milieu acide.

9. Procédé selon la revendication 7, **caractérisé en ce que** la préparation du 2,5-diméthylhexane-2,5-dihydroperoxyde est réalisée par réaction du 2,5-diméthyl-1,5-hexadiène avec le peroxyde d'hydrogène en milieu acide.

10. Procédé selon l'une des revendications 6 à 9, **caractérisé en ce que** l'hydroperoxyde solide est mis en suspension entre l'étape b) et l'étape c), dans un liquide de lavage et la suspension obtenue est filtrée par le dispositif de filtration (5) agencé dans le fond du récipient (2) avec retenue de l'hydroperoxyde solide dans le réacteur.

11. Procédé selon l'une des revendications 6 à 10, **caractérisé en ce que** l'hydroperoxyde de l'étape c) est dissous dans un solvant avant de le faire réagir avec l'agent d'alkylation, l'agent d'acylation ou le composé carbonylé.

12. Procédé selon l'une des revendications 6 à 11, **caractérisé en ce que** l'agent d'alkylation de l'étape c) est un alcool tertiaire et la réaction est effectuée en présence d'un acide fort.

13. Procédé selon la revendication 12, **caractérisé en ce que** l'agent d'alkylation est un alcool tertiaire de la série du t-butanol, du t-amylalcool et du cumylalcool.

14. Procédé selon l'une des revendications 6 à 11, **caractérisé en ce que** l'agent d'acylation de l'étape c) est choisi parmi les chlorures d'acide carboxylique, les anhydrides d'acide carboxylique et les chloroformiates.

15. Procédé selon la revendication 14, **caractérisé en ce que** l'agent d'acylation est un chlorure d'acide carboxylique de la série du chlorure d'acétyle, le chlorure d'isobutyryle, le chlorure de pivaloyle, le chlorure de 2-éthylhexanoyle, le chlorure de 3,5,5-tri-méthylhexanoyle, le chlorure de néodécanoyle et le chlorure de benzoyle.

16. Procédé selon la revendication 14, **caractérisé en ce que** l'agent d'acylation est un chloroformiate de la série du chloroformiate d'isopropyle, du chloroformiate de n-butyle, du chloroformiate de s-butyle, du chloroformiate de 2-éthylhexyle, du chloroformiate de myristyle, du chloroformiate de cétyle, du chloroformiate de cyclohexyle, et du chloroformiate de 4-t-butylcyclohexyle.

17. Procédé selon l'une des revendications 6 à 11, **caractérisé en ce que** le composé carbonylé est l'acétone.
